# FASCICULE DE BREVET EUROPEEN

(11) **EP 3 095 852 B1**
(45) Date de publication et mention de la délivrance du brevet: **24.06.2020**
(21) Numéro de dépôt: 16170706.2
(22) Date de dépôt: 20.05.2016
(51) Int. Cl.: C12M 1/38, C12M 1/02, C12Q 3/00

(54) **PROCEDE ET DISPOSITIF DE CONTROLE DE LA TEMPERATURE D'UNE REACTION EXOTHERMIQUE OU ENDOTHERMIQUE**
VERFAHREN UND VORRICHTUNG ZUR TEMPERATURKONTROLLE EINER EXO- ODER ENDOTHERMISCHEN REAKTION
METHOD AND DEVICE FOR CONTROLLING THE TEMPERATURE OF AN EXOTHERMAL OR ENDOTHERMAL REACTION

(30) Priorité: 22.05.2015 FR 1554603
(43) Date de publication de la demande: 23.11.2016
(73) Titulaire: Oeno Concept, 51530 Mardeuil (FR)
(72) Inventeur: TAIDI, Behnam, 92160 Antony (FR); HUSSENET, Clément, 51370 Les Mesneux (FR); POISOT, Maxime, 51530 Saint Martin D'Ablois (FR)
(74) Mandataire: Cabinet Bleger-Rhein-Poupon

(56) Documents cités:
- EP-A2- 0 089 225
- WO-A1-99/46209
- WO-A1-2011/140649
- WO-A1-2015/018907
- FR-A1- 2 606 514
- US-B2- 8 283 159
- RICHARD BIENER ET AL: "Calorimetric control of the specific growth rate during fed-batch cultures of", JOURNAL OF BIOTECHNOLOGY, ELSEVIER SCIENCE PUBLISHERS, AMSTERDAM, NL, vol. 160, no. 3, 9 mars 2012 (2012-03-09), pages 195-201, XP028431929, ISSN: 0168-1656, DOI: 10.1016/J.JBIOTEC.2012.03.006 [extrait le 2012-03-17]
- YUZGEC U ET AL: "On-line evolutionary optimization of an industrial fed-batch yeast fermentation process", ISA TRANSACTIONS, INSTRUMENT SOCIETY OF AMERICA. PITTSBURGH, US, vol. 48, no. 1, 1 janvier 2009 (2009-01-01), pages 79-92, XP025800523, ISSN: 0019-0578, DOI: 10.1016/J.ISATRA.2008.09.001 [extrait le 2008-10-11]
- AIBA S ET AL: "FED BATCH CULTURE OF SACCHAROMYCES-CEREVISIAE A PERSPECTIVE OF COMPUTER CONTROL TO ENHANCE THE PRODUCTIVITY IN BAKERS YEAST CULTIVATION", BIOTECHNOLOGY AND BIOENGINEERING, WILEY & SONS, HOBOKEN, NJ, US, vol. 18, no. 7, 1 janvier 1976 (1976-01-01), pages 1001-1016, XP002507590, ISSN: 0006-3592, DOI: 10.1002/BIT.260180712
- Mustafa Türker: "Measurement of metabolic heat in a production-scale bioreactor by continuous and dynamic calorimetry", Chemical Engineering Communications, vol. 190, no. 5-8, 1 May 2003 (2003-05-01) , pages 573-598, XP055484718, US ISSN: 0098-6445, DOI: 10.1080/00986440302130
- VALENTINOTTI S ET AL: "Optimal operation of fed-batch fermentations via adaptive control of overflow metabolite", CONTROL ENGINEERING PRACTICE, PERGAMON PRESS, OXFORD, GB, vol. 11, no. 6, 1 June 2003 (2003-06-01), pages 665-674, XP027129815, ISSN: 0967-0661 [retrieved on 2003-06-01]

## Description

La présente invention concerne un procédé de contrôle de la température d'une réaction chimique ou biologique au cours de laquelle de la chaleur est produite ou consommée. Plus particulièrement, on cherche à maintenir la température à un niveau constant malgré la production ou la consommation de chaleur due au déroulement de ladite réaction.

Le procédé selon l'invention trouve une application particulièrement intéressante dans le domaine de la production de microorganismes d'intérêt.

Plus particulièrement, il est recherché, au moyen de la présente invention, une méthode destinée à permettre la production de micro-organismes d'intérêt, notamment des levures, de préférence en grande quantité au niveau industriel. Pour cela, une maîtrise de la température est nécessaire.

Il est déjà connu, dans l'état de la technique, certains procédés destinés à permettre un contrôle des processus fermentaires effectués par des microorganismes, notamment par des levures.

Le processus de fermentation consiste en un procédé biotechnologique qui est largement utilisé au sein des industries pharmaceutiques et alimentaires. Grâce au génie fermentaire, il est possible de produire notamment des levures d'intérêt et/ou de faire synthétiser, par lesdites levures, des molécules d'intérêt.

De ce fait, de nombreux travaux portent sur la régulation des procédés impliquant des microorganismes, et notamment des levures.

Ainsi, le document de brevet EP 0 089 225 décrit un dispositif et une méthode de régulation d'une fermentation d'hydrates de carbone destinée à permettre la production d'alcool.

Dans cette méthode, le développement de la levure est régulé par le contrôle de la valeur du pH du milieu dans lequel la levure croît, autrement dit le moût de fermentation.

Plus particulièrement, il est prévu des détecteurs de pH dans la cuve de fermentation, et des moyens de contrôle comparant le pH mesuré dans la cuve à un pH de référence. Si le pH mesuré est inférieur audit pH de référence à un instant donné, le dispositif comporte des moyens permettant une injection d'oxygène dans la cuve de fermentation.

La présence d'une boucle dans laquelle circule un liquide de refroidissement est également prévue.

Toutefois, le procédé décrit ici ne permet pas une régulation et un contrôle précis de la température directement dans la cuve de fermentation.

On connaît encore, par le document FR 2 563 531 un procédé et un appareillage pour la fermentation. Dans ce procédé, la température est contrôlée pendant la fermentation alcoolique, aux alentours d'une température de 28°C considérée comme étant la température optimale.

Afin de réguler la température, l'appareillage comporte un récipient extérieur pourvu intérieurement d'un sac étanche dans lequel sont placés la matière devant fermenter, notamment les levures, et le milieu de fermentation.

L'espace entre ledit récipient et ledit sac constitue une chambre dans laquelle est mis en circulation un fluide réfrigérant, par exemple de l'eau, de sorte à ce que le sac prenne le volume de son contenu. On contrôle ainsi, au moyen du fluide qui circule dans ladite chambre, la température du milieu de fermentation.

Toutefois, un tel procédé est compliqué à mettre en œuvre. En outre, l'échange thermique est limité par la conductivité thermique du matériel dans lequel est fabriqué le sac. De ce fait, ce procédé ne permet pas d'aboutir à un contrôle précis de la température du milieu de fermentation.

On connait également, du document de brevet WO 2015/018907 un appareillage pour la production d'aliments et de boissons fermentés dans un premier réservoir comportant un substrat de départ.

Ledit appareillage comporte, en outre, une unité de contrôle de la température au cours de la phase de fermentation. Cette unité incorpore un matériel de stockage de chaleur latente dans un second réservoir. Ledit matériel peut se présenter sous forme de particules par exemple, et est couplé uniquement thermiquement au substrat à fermenter, sans entrer en contact avec celui-ci. Le matériel de stockage de chaleur peut également être séparé du substrat par une cloison dans un échangeur de chaleur à plaques ou à tubes.

Cet appareillage nécessite donc l'utilisation d'un matériel particulier, particules à stockage de chaleur latente par exemple, car il est nécessaire d'éviter toute contamination du produit fini par le matériel de stockage à chaleur latente.

Ainsi, là encore, le dispositif est complexe.

Il est encore connu, du document de brevet FR 2 606 514 un procédé dans lequel on détermine, à intervalles de temps réguliers, au moins un paramètre de fermentation. Ce paramètre peut être la densité du milieu de fermentation, son indice de réfraction, ou encore le volume de dioxyde de carbone (CO₂) produit au cours de la fermentation alcoolique opérée par la levure.

L'objectif ici est de prédire l'évolution de la fermentation alcoolique et de la contrôler. Ce contrôle peut s'effectuer notamment au moyen du suivi de l'évolution de la température ou de la vitesse de dégradation des sucres, par une unité de traitement qui reçoit les paramètres mesurés et les transfère, via une unité périphérique, à une unité électronique de commande. Cette dernière pilote les actionneurs régulant les paramètres de fermentation.

Par conséquent, les paramètres de croissance d'une souche doivent être connus au préalable.

En outre, l'ensemble des procédés décrits dans ces documents s'appliquent au suivi de paramètres au cours de la fermentation alcoolique opérée par des levures, et ce afin d'optimiser cette étape de fermentation.

Or, dans la présente invention, l'un des objectifs visés est la production de biomasse, notamment à partir de levures, en grande quantité et, à cet effet, on cherche à éviter que la levure entre dans un processus de fermentation (métabolisme sans la consommation de l'oxygène), soit totalement, soit partiellement pendant une durée plus ou moins longue. En effet, pendant la fermentation, la production de biomasse diminue substantiellement.

Ainsi, il est apparu que les solutions existantes dans l'état de la technique ne sont pas adaptées à une optimisation de la production de la biomasse.

En outre, la plupart de ces solutions proposées sont compliquées et/ou coûteuses à mettre en œuvre et/ou nécessitent des études préliminaires.

L'invention offre la possibilité de pallier les divers inconvénients de l'état de la technique en proposant notamment un procédé simple à mettre en œuvre et innovant de production de biomasse à partir de microorganismes, en particulier des levures, dans lequel la température du milieu de croissance des microorganismes est contrôlée et maintenue constante en permanence dans une gamme de température optimale pour la production de biomasse en quantité importante.

Plus précisément, de manière originale, la température du milieu réactionnel est régulée par l'apport de substrat ou de nutriments, de préférence dans un milieu présentant une température basse.

Plus généralement, le procédé selon l'invention peut être appliqué à tout type de réaction, chimique ou biologique, lorsque ladite réaction est exothermique, entraînant de ce fait une variation de température au sein du milieu dans lequel elle s'effectue.

Ainsi, la présente invention concerne un procédé de contrôle de la température d'une réaction exothermique, productrice de chaleur, ladite réaction ayant lieu dans un milieu réactionnel et faisant intervenir des cellules animales et/ou végétales et/ou des microorganismes, procédé dans lequel :
- on inocule ledit milieu réactionnel avec lesdites cellules ou lesdits microorganismes ;
- on permet à la réaction exothermique d'avoir lieu dans ledit milieu réactionnel ;
- on mesure une variation de température due à la réaction exothermique, cette variation étant une augmentation ;
- on ajoute progressivement audit milieu réactionnel, au cours de ladite réaction, au moins une source externe comprenant un substrat et/ou un réactif ;
ledit procédé étant caractérisé par le fait que l'on maintient, dans le milieu réactionnel, une température sensiblement constante en contrôlant au moins l'alimentation en source externe et la température de ladite source externe, l'alimentation en source externe et la température de cette dernière étant aptes à compenser la variation de la température mesurée due à la réaction exothermique.

Selon d'autres caractéristiques du procédé de l'invention :
- en plus de contrôler l'alimentation et la température de la source externe, on protège le milieu réactionnel de toute influence externe en termes d'apport ou de déperdition de chaleur ;
- dans le cas d'une réaction exothermique productrice de chaleur, on maintient une température sensiblement constante dans le milieu réactionnel en ajoutant une source externe présentant une température basse apte à compenser la production de chaleur engendrée par ladite réaction exothermique ;
- on maintient une température sensiblement constante dans le milieu réactionnel en contrôlant, en plus de la température de ladite source externe ajoutée audit milieu réactionnel, au moins la concentration en substrat et/ou en réactif dans ledit milieu réactionnel.

Dans le cadre d'une réaction exothermique, et dans un mode de réaction particulier du procédé de l'invention, ladite réaction exothermique permet la production de biomasse à partir de microorganismes, procédé dans lequel :
- on inocule un milieu réactionnel approprié, consistant en un milieu de culture liquide, avec lesdits microorganismes afin que ces derniers se multiplient dans ledit milieu de culture ;
- on mesure l'augmentation de la température due à la production de chaleur engendrée par la réaction exothermique de production de biomasse ;
- on ajoute progressivement, audit milieu de culture, au moins une source externe liquide comprenant un substrat carboné pour lesdits microorganismes ;
- on maintient, tout au long de la réaction de production de biomasse, une température sensiblement constante dans ledit milieu de culture en ajoutant une source externe liquide présentant une température basse apte à compenser ladite production de chaleur engendrée par ladite réaction de production de biomasse.

Selon des caractéristiques additionnelles du procédé de l'invention :
- lesdits microorganismes consistent en des levures *Saccharomyces cerevisiae ;*
- le milieu de culture liquide est un milieu de culture hydroalcoolique ;
- le milieu de culture hydroalcoolique est supplémenté en glucides ;

La présente invention concerne également un dispositif pour la mise en œuvre du procédé de contrôle de la température d'une réaction exothermique, comportant :
- au moins une cuve de propagation comprenant un milieu réactionnel inoculé avec des cellules animales et/ou végétales et/ou des microorganismes et au moins des moyens de mesure de la température de réaction à l'intérieur de ladite cuve de propagation ;
- un centre de collecte des données mesurées par lesdits moyens et de contrôle de ladite température de réaction ;
- au moins une cuve de stockage comprenant une source externe, qui comprend un substrat et/ou un réactif, et étant reliée à ladite cuve de propagation par un moyen de transfert apte à être activé par ledit centre de contrôle pour acheminer progressivement la source externe depuis ladite cuve de stockage vers ladite cuve de propagation ;
ledit dispositif étant caractérisé par le fait que ladite cuve de propagation est isolée thermiquement et que ladite cuve de stockage est thermorégulée de manière à contrôler la température de la source externe transférée vers ladite cuve de propagation, ladite cuve étant réfrigérée via des moyens de refroidissement et maintenue à basse température au moyen d'une double enveloppe ou d'un circuit de refroidissement, l'ajout de la source externe à une température basse étant apte à compenser la production de chaleur et une augmentation de température dans la cuve de propagation.

D'autres caractéristiques et avantages de l'invention ressortiront de la description détaillée qui va suivre des modes de réalisation non limitatifs de l'invention, en référence à :
- la figure 1 représentant schématiquement un mode de réalisation particulier d'une partie du dispositif pour la mise en œuvre du procédé selon la présente invention, notamment la cuve de stockage et la cuve de propagation, ainsi qu'un récipient de réhydratation des levures ;
- la figure 2 représentant schématiquement un mode de réalisation particulier et détaillé du dispositif pour la mise en œuvre du procédé selon la présente invention.

Le procédé selon la présente invention a pour objectif principal de permettre une régulation de la température d'une réaction exothermique ayant lieu dans un milieu réactionnel, ladite réaction faisant intervenir des cellules animales et/ou végétales et/ou des microorganismes.

Dans la nature, il existe trois types de réactions chimiques :
- les réactions exothermiques qui génèrent de la chaleur et entraînent une augmentation de la température du milieu dans lequel s'effectue la réaction ;
- les réactions endothermiques qui absorbent de la chaleur et entraînent une diminution de la température du milieu réactionnel;
- les réactions athermiques qui ne produisent, ni ne consomment, de chaleur, n'entraînant ainsi aucun changement dans la température du milieu.

Dans le présent procédé, la régulation de la température d'une réaction exothermique, est effectuée, de manière originale, au moyen du contrôle de l'apport d'une source externe dans le milieu réactionnel.

Ladite source externe consiste, par exemple, en un réactif ou en un substrat, et on alimente le milieu réactionnel, avec ladite source, de manière contrôlée et à une température contrôlée, et ce en fonction du type de réaction.

En d'autres termes, le procédé selon l'invention propose un contrôle de l'alimentation en source externe et de la température de ladite source, en lien avec la production de chaleur au sein du milieu réactionnel.

L'ajout de la source externe est donc effectué en fonction d'une augmentation de température dans ledit milieu, et cela permet un maintien de la concentration en substrat dans la bonne gamme pour un déroulement optimisé de la réaction, à une température sensiblement constante.

De manière plus spécifique, lors de la première étape du procédé, on inocule le milieu réactionnel. Ce dernier est généralement un milieu de culture liquide placé dans une cuve, et il est inoculé avec des cellules animales et/ou végétales et/ou des microorganismes.

De préférence, le milieu est inoculé avec un seul type de cellules, par exemple des microorganismes, ces derniers pouvant notamment consister en des levures.

La réaction exothermique peut ensuite se dérouler au sein dudit milieu réactionnel, et va entraîner une production de chaleur, qui va se traduire par une augmentation de la température de réaction au sein du milieu.

Ces variations de température du milieu réactionnel vont être mesurées tout au long de la réaction exothermique, notamment au moyen de sondes ou de capteurs de température.

Afin de compenser ces variations de température dues à la production de chaleur, tout au long de la réaction, on va ajouter progressivement une source externe, contenant du substrat ou un réactif, à une température adéquate de sorte à maintenir une température sensiblement constante dans le milieu réactionnel.

En d'autres termes, la détection d'une variation de température est utilisée comme signal pour déclencher l'ajout d'une quantité adéquate de source externe au milieu réactionnel, en général un certain volume, la température de la source externe étant appropriée pour un maintien dudit milieu réactionnel à température optimale. On arrête ensuite l'ajout, puis on le reprend dès lors qu'une nouvelle variation de température dans le milieu réactionnel est mesurée, en sorte que la température dudit milieu est toujours sensiblement constante et optimale.

Une température sensiblement constante dans un milieu réactionnel, au sens de la présente invention, signifie que la température au sein du milieu réactionnel peut varier autour d'une valeur considérée comme optimale, dans une gamme de températures comprises entre une valeur minimale et une valeur maximale acceptables.

Ainsi, par exemple, dans le cas de figure où la réaction est la plus efficace à 28°C, la température du milieu réactionnel peut varier de plus ou moins 3°C, de préférence plus ou moins 2°C, et plus préférentiellement encore de plus ou moins 1°C autour de cette valeur de 28°C.

Par conséquent, dans le cadre de cet exemple, la température du milieu réactionnel est maintenue sensiblement constante dans une plage allant de 25 à 31°C, de préférence entre 26 et 30°C, et de préférence encore entre 27 et 29°C.

Bien évidemment, cette définition s'applique quelle que soit la température T considérée comme optimale de la réaction.

Ainsi, de manière générale, considérant une température optimale de T°C, la température dans le milieu réactionnel est maintenue sensiblement constante entre T-3 et T+3°C, de préférence entre T-2 et T+2°C, de préférence entre T-1 et T+1°C, par le contrôle de l'alimentation en source externe.

En outre, de manière avantageuse, on protège le milieu réactionnel de toute influence externe en termes d'apport ou de déperdition de chaleur. Pour ce faire, il est envisageable d'isoler thermiquement une cuve dans laquelle serait placée ledit milieu.

Par ce biais, on évite que la variation de température du milieu ne soit due à des facteurs externes, ce qui présente l'avantage de faciliter le contrôle de la température dudit milieu par l'alimentation en source externe. En effet, dans ce cas, les variations de température du milieu ne sont dues qu'à la production de chaleur engendrée par la réaction.

En ce qui concerne à présent la source externe, celle-ci peut consister en du milieu réactionnel neuf, ou milieu de culture.

Ainsi, dans le présent procédé, le mode de culture qui est choisi est dit « de culture en discontinu alimentée » ou culture en « fed batch » en anglais : dans ce mode de culture, le volume de départ est réduit, ce qui permet notamment un démarrage rapide de la multiplication des microorganismes. Du milieu est ajouté de manière progressive, et sans soutirage, de sorte que le volume dans la cuve augmente au cours de la culture.

La culture est arrêtée lorsque le volume de milieu de culture atteint une certaine valeur.

Généralement, les réactions opérées par des systèmes biologiques, notamment par des cellules végétales ou animales ou encore par des microorganismes, sont des réactions exothermiques et donc productrices de chaleur.

Dans ce cas de figure, on mesure une augmentation de la température dans le milieu et on maintient une température sensiblement constante dans ledit milieu en ajoutant une source externe, consistant par exemple en du milieu neuf, dont la température est basse.

De préférence, la température de ladite source externe est inférieure à celle du milieu réactionnel et est apte à compenser l'augmentation de la température due à la production de chaleur engendrée par la réaction exothermique.

Dans un mode de réalisation avantageux, la source externe est apportée à une température comprise entre - 5 et 10°C, de préférence entre 0° et 5°C, de préférence encore cette température est de 0°C ou proche de 0°C.

La température de la source externe peut avantageusement être corrélée avec sa concentration en substrat et/ou en réactif. Ainsi, plus la concentration en substrat et/ou en réactif est élevée, plus la température de la source externe sera froide.

En effet, lorsque la concentration en réactif ou en substrat dans la source externe est élevée, celle-ci va entraîner un apport important dans le milieu réactionnel. De ce fait, les cellules ou les microorganismes auront à leur disposition une quantité élevée de substrat ou de réactif pour réaliser la réaction exothermique, entraînant une augmentation rapide de la température au sein dudit milieu. Une température très basse de la source externe permet donc de compenser de manière optimale une activité importante des cellules ou microorganismes.

Pour récapituler, dans le cadre d'une réaction exothermique productrice de chaleur, dès que l'on détecte une augmentation de la température dans le milieu réactionnel, on ajoute un certain volume de source externe à basse température, inférieure à la température dudit milieu.

De manière générale, la température de la source externe peut dépendre de l'énergie libérée par la réaction et/ou de l'énergie du substrat ou des réactifs potentiellement disponible et/ou de leurs concentrations.

Une application particulière et intéressante du présent procédé consiste à maintenir une température constante pour favoriser la production de biomasse à partir de cellules ou de microorganismes.

En effet, une partie de la chaleur libérée lors des réactions exothermiques est transformée en énergie utilisable par les cellules ou les microorganismes, sous forme d'ATP (Adénosine Tri-Phosphate). Cette ATP est ensuite utilisée par les cellules ou les microorganismes dans la production de biomolécules et notamment de biomasse.

En d'autres termes, l'énergie produite sous forme d'ATP peut être utilisée par des microorganismes, par exemple des levures, pour leur propre multiplication.

De ce fait, le procédé selon la présente invention est tout particulièrement indiqué pour la production de microorganismes d'intérêt, en quantité importante, par une multiplication optimisée de ces derniers.

De manière préférentielle, les microorganismes que l'on souhaite produire en grande quantité, sous forme de biomasse, consistent en des levures.

Les levures obtenues au moyen du présent procédé peuvent notamment être utilisées, par la suite, pour permettre la fermentation dans l'optique d'obtenir des boissons alcoolisées.

Toutefois, une telle application des levures produites ne doit pas être considérée comme étant limitative et d'autres applications peuvent être envisagées.

En particulier, les levures, ou autres cellules ou microorganismes, utilisés ou produits lors de la mise en œuvre du présent procédé, peuvent avoir une application dans la production de molécules ou de métabolites d'intérêt, notamment dans les industries pharmaceutiques ou agroalimentaires.

Les levures sont des microorganismes ayant la capacité de métaboliser des composés carbonés, notamment le glucose, via deux voies différentes :
- la voie fermentaire : le glucose est transformé en dioxyde de carbone et en éthanol, avec production d'énergie sous forme de chaleur.
- la voie respiratoire : en présence d'oxygène, autrement dit en aérobiose, les levures transforment le glucose en dioxyde de carbone et en eau, avec production d'énergie sous forme de chaleur.

Les réactions de dégradation des nutriments par les levures, notamment des nutriments carbonés comme par exemple le glucose, sont donc des réactions exothermiques.

Ainsi, lors de la croissance et de la multiplication de microorganismes, notamment de levures, dans un milieu de culture approprié, la température de ce milieu est susceptible d'augmenter de manière non négligeable.

Or, dans l'optique d'une production optimisée de microorganismes, il est nécessaire de maintenir une température sensiblement constante dans le milieu de culture, notamment parce que les levures ne sont pas des organismes thermorésistants.

Le procédé selon la présente invention est donc particulièrement intéressant dans le cadre de la production de levures, car il permet une régulation ciblée de la température dans leur milieu de culture.

En outre, en mettant en œuvre ledit procédé, on peut favoriser la dégradation des sucres majoritairement via la voie respiratoire, qui permet une optimisation de la production de biomasse.

En effet, lorsque les levures métabolisent les sucres par la voie fermentaire, la production de biomasse est moins importante que lors de la respiration. De ce fait, il convient de favoriser un métabolisme des sucres par la respiration aérobie.

Lorsque la concentration en glucose est trop élevée, la consommation d'oxygène s'arrête et les levures ne métabolisent plus le glucose par voie respiratoire mais par la voie de la fermentation alcoolique. C'est ce qu'on appelle l'effet Crabtree qui correspond à une inhibition du métabolisme respiratoire du glucose et ce même en présence d'oxygène.

En d'autres termes, la concentration de glucose dans le milieu de culture a une influence sur le choix métabolique des levures, et un milieu de culture riche en glucose inhibe le métabolisme respiratoire.

Aussi, la concentration en glucose dans le milieu de culture doit être préférentiellement maintenue basse et contrôlée tout au long du procédé de production de la biomasse. Cette concentration est de préférence inférieure à une concentration de 10 g/L.

Au moyen du procédé selon l'invention, et notamment de l'ajout progressif de substrat, il est aisé de contrôler la concentration en substrat, notamment en glucose, dans le milieu réactionnel.

Pour ce qui est des microorganismes cultivés dans l'optique de produire de la biomasse en quantité importante, ceux-ci consistent avantageusement en des levures appartenant au genre *Saccharomyces.*

Plus particulièrement encore, ces levures appartiennent à *Saccharomyces cerevisiae* et, avantageusement, l'inoculum de départ consiste en de la levure sèche active (LSA).

Dans un mode de réalisation particulier et avantageux, le milieu de culture utilisé dans le présent procédé pour la multiplication de microorganismes, lorsque ceux-ci sont des levures, consiste en un milieu hydroalcoolique.

Lorsque ledit milieu de culture est un milieu hydroalcoolique, celui-ci peut être avantageusement enrichi au moyen d'ajout de nutriments tels que notamment des glucides, ou carbohydrates, qui regroupent les composés organiques contenant un groupe carbonyle (aldéhyde ou cétone) et au moins deux groupes hydroxyle (-OH).

Plus précisément, ledit milieu de culture peut être enrichi au moyen de l'ajout de saccharose.

En effet, comme déjà évoqué précédemment, le présent procédé permet avantageusement une optimisation de la production de levures qui seront ensuite utilisées dans la mise en œuvre de procédés fermentaires destinés à permettre la production de boissons alcoolisées.

Aussi, le fait de produire en amont la levure sur un milieu correspondant à son milieu de destination, en l'occurrence un milieu hydroalcoolique, permet une meilleure acclimatation de ladite levure et une fermentation ultérieure optimisée.

En outre, l'utilisation d'un milieu hydroalcoolique, lors de la mise en œuvre du présent procédé, permet d'éviter la préparation d'un milieu synthétique. En effet, une telle préparation peut être source d'erreur, du fait des nombreuses opérations nécessaires (pesées, volume, etc.).

Un autre avantage de l'utilisation d'un milieu hydroalcoolique réside dans le fait que celui-ci est particulièrement économique, en comparaison avec un milieu complètement synthétique.

Toutefois, un tel mode de réalisation ne doit pas être compris comme étant limitatif de l'invention, et il est également envisageable que le milieu de culture utilisé dans le présent procédé ne contienne pas d'alcool.

Dans le procédé selon la présente invention, il est également envisageable, et avantageux, de contrôler notamment la concentration en substrat, ou en nutriments, dans le milieu réactionnel.

Le débit d'alimentation, ou la vitesse d'alimentation, de la source externe, contenant les nutriments nécessaires à la croissance des levures, peut être adapté et contrôlé pour obtenir la concentration voulue en nutriments dans le milieu réactionnel.

Ainsi, à partir de cette concentration, il est possible d'estimer la consommation de substrat par la levure, notamment de glucose, et donc la chaleur dégagée lors de la multiplication des levures.

Une telle estimation peut permettre de déclencher l'apport de milieu de culture, celui-ci pouvant être refroidi, au moment opportun pour maintenir la température de réaction dans une gamme de température considérée comme optimale, de préférence sensiblement constante.

Dans le cadre de la production de levures en grande quantité, le présent procédé est particulièrement intéressant. Par un contrôle constant de la température de réaction, ledit procédé permet, à tout moment lors de la multiplication des levures, de maintenir une population importante et de diminuer au maximum le temps de remultiplication des levures.

En outre, il est également envisageable de déclencher l'apport en source externe, notamment du milieu de culture neuf, lorsque la température mesurée dans le milieu réactionnel atteint un plateau de température ou diminue, ce qui peut correspondre à un ralentissement ou un arrêt de la multiplication des levures.

Un mode de réalisation particulier du procédé selon la présente invention a été décrit ci-dessus, dans lequel le microorganisme cultivé consiste préférentiellement en une levure, notamment du type *Saccharomyces cerevisiae,* et dans lequel la réaction de production de biomasse, dont la température est contrôlée, est une réaction exothermique productrice de chaleur.

De manière plus générale, le procédé décrit ici est applicable à toutes les réactions chimiques ou biologiques au cours desquelles au moins une source externe, comprenant au moins un réactif et/ou au moins un substrat, est apportée à la réaction de manière progressive.

Ainsi, avantageusement, en contrôlant l'alimentation et la température dudit réactif et/ou substrat, il est possible de contrôler indirectement et d'optimiser la température de la réaction qui est en cours.

Le procédé selon l'invention est également applicable pour la production de tous types de cellules, qu'il s'agisse de cellules animales, végétales ou microbiennes.

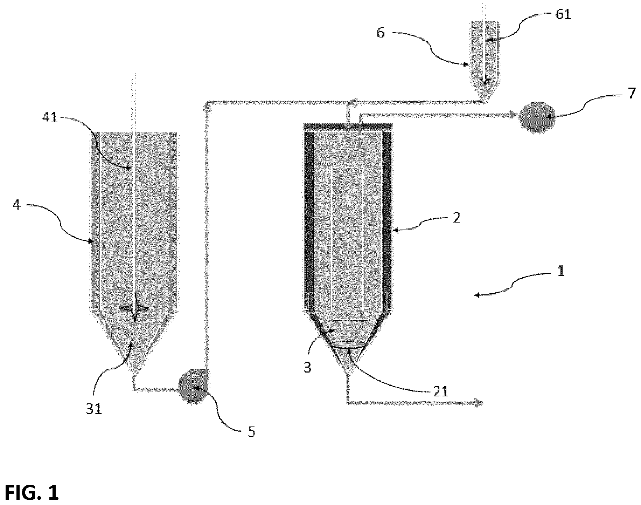

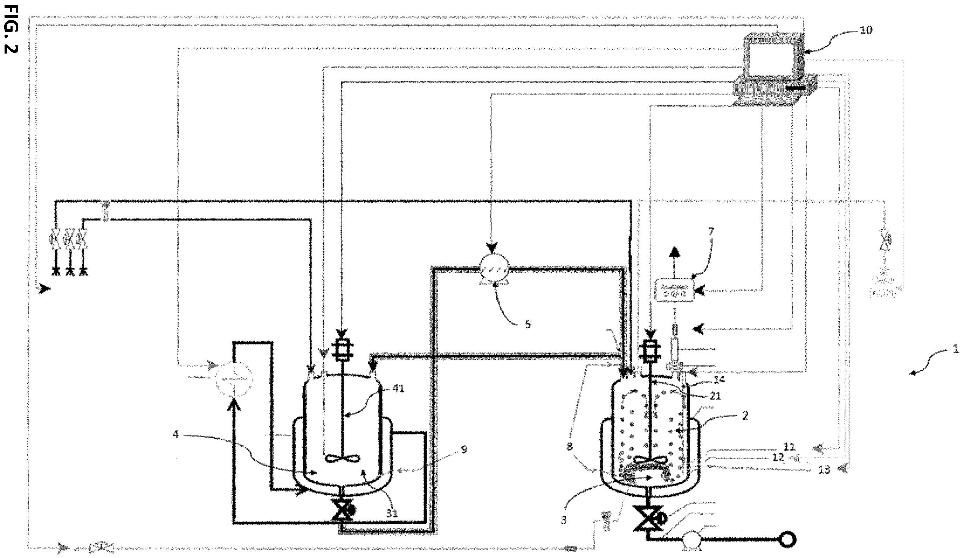

La présente invention est également relative à un dispositif 1 pour la mise en œuvre du procédé décrit précédemment, celui-ci étant représenté sur les figures 1 et 2 annexées.

Ledit dispositif 1 comporte, de manière préférentielle :
- au moins une cuve de propagation 2 comprenant un milieu réactionnel 3 inoculé avec des cellules animales et/ou végétales et/ou des microorganismes et au moins des moyens de mesure de la température de réaction à l'intérieur de ladite cuve de propagation 2 ;
- un centre de collecte des données mesurées et de contrôle de ladite température de réaction ;
- au moins une cuve de stockage 4 comprenant une source externe 31, qui comprend un substrat et/ou un réactif pour les cellules ou les microorganismes, ladite cuve de stockage 4 étant reliée à ladite cuve de propagation 2 par un moyen de transfert 5 apte à être activé par ledit centre de contrôle pour permettre un acheminement progressif de la source externe 31 depuis ladite cuve de stockage 4 vers ladite cuve de propagation 2.

Le dispositif selon l'invention est particulier en ce que, d'une part, ladite cuve de propagation 2 est isolée thermiquement et que, d'autre part, ladite cuve de stockage 4 est thermorégulée de manière à contrôler la température de la source externe 31 transférée vers ladite cuve de propagation 2.

Le fait que la cuve de propagation 2 soit isolée thermiquement est particulièrement intéressant car cela permet d'éviter, par exemple, toute déperdition de chaleur pour ce qui est d'une réaction exothermique.

En d'autres termes, cela facilite le contrôle de la température réactionnelle et le maintien de celle-ci à un niveau constant par l'ajout de la source externe 31 à une température adéquate. En outre, ladite cuve 2 est préférentiellement fermée, toujours dans le but d'éviter des déperditions de chaleur à l'extérieur de ladite cuve 2.

Pour ce qui est de la température de la source externe 31 dans la cuve de stockage 4, celle-ci est contrôlée au moyen d'une régulation de la température au sein de ladite cuve 4 contenant ladite source externe 31, cette dernière devant être ajoutée de manière incrémentale dans la cuve de propagation 2.

Ainsi, dans le cas où la réaction est une réaction exothermique de production de biomasse, la cuve de stockage 4 est réfrigérée via des moyens de refroidissement, et maintenue à basse température au moyen, par exemple, d'une double enveloppe ou d'un circuit de refroidissement. De ce fait, l'ajout de la source externe 31 à une température basse permet de compenser la production de chaleur et donc une augmentation de température dans la cuve de propagation 2.

La partie supérieure de la cuve de stockage 4 est indifféremment ouverte ou fermée. Cette cuve 4 peut également être équipée, avantageusement, de moyens d'agitation 41 permettant notamment de maintenir une température homogène de la source externe 31 contenue à l'intérieur de ladite cuve 4.

Le dispositif 1 selon l'invention comporte en outre un moyen de transfert 5 de la source externe 31 depuis la cuve de stockage 4 vers la cuve de propagation 2.

De préférence, ce moyen de transfert consiste en une pompe d'alimentation 5 connectée à ladite cuve de stockage 4 pour permettre un transfert de la source externe 31 depuis ladite cuve de stockage 4 vers la cuve de propagation 2.

Pour contrôler ce transfert, ladite pompe d'alimentation 5 peut être avantageusement reliée à un centre de collecte des données et de contrôle 10, visible sur la figure 1, et permettant une activation de ladite pompe 5 au moment opportun, lorsqu'une augmentation de la température est mesurée par les sondes par exemple, pour permettre un refroidissement du milieu réactionnel 3 au sein de la cuve 2 au moyen de l'ajout d'un certain volume de source externe 31.

Le transfert de source externe 31 entre les deux cuves de stockage 4 et de propagation 2 peut également s'effectuer par gravité.

Dans tous les cas, le moyen de transfert sera toujours isolé un maximum afin d'éviter tout changement de température entre la cuve de stockage 4 et la cuve de propagation 2.

Dans le cas particulier où l'application visée est la propagation de levures, déjà évoquée, le centre de collecte des données et de contrôle 10 peut être configuré pour déterminer la quantité de glucose (par exemple) consommée en fonction de l'augmentation de température qui est détectée. Ainsi, ledit centre de contrôle 10 permet ensuite l'ajout de volume adéquat de source externe pour remplacer la quantité de glucose consommée, par exemple en fonction de la souche de levure, de l'inoculum de départ, etc.

En ce qui concerne à présent ladite cuve de propagation 2, il a déjà été évoqué qu'elle comporte au moins des moyens de mesure de la température de réaction. Ces moyens peuvent notamment consister en des sondes ou capteurs 8 mesurant la température du milieu réactionnel et enregistrant les variations de sa température. Une sonde de mesure de la température 9 peut également être présente au niveau de la cuve de stockage 4.

En plus de la (ou des) sonde (s) 8 de contrôle de la température, ladite cuve 2 peut également comporter une pluralité d'autres sondes ou capteurs de mesure et d'enregistrement.

Ainsi, par exemple, ladite cuve 2 peut être équipée d'une sonde de mesure et d'enregistrement du pH 11 du milieu réactionnel 3 et/ou une sonde de mesure d'oxygène dissous 12 et/ou une sonde de mesure de biomasse 13 et/ou une sonde de niveau 14.

Ladite cuve 2 comporte également avantageusement des moyens d'agitation 21 pouvant consister notamment soit en des moyens mécanique d'agitation, soit en des moyens d'agitation par gaz ou air (« gaz-lift » ou « air-lift » en anglais), notamment dans le cas d'une réaction de production de biomasse par des levures.

Avantageusement, la cuve de propagation 2 présente un volume égal à environ deux fois la capacité requise par l'utilisateur pour produire de la biomasse, par exemple.

Ainsi, dans l'hypothèse où l'utilisateur souhaite obtenir un volume de culture de 500L par jour, ladite cuve de propagation doit avoir un volume utile d'au moins 1000L.

Le dispositif 1 selon l'invention peut également comporter un récipient 6 de réhydratation des levures, incorporant des moyens d'agitation 61 et des moyens de chauffage et/ou des moyens de refroidissement, et représenté sur la figure 1. Ce récipient 6 peut être indifféremment ouvert ou fermé.

La présence de ce récipient 6 est particulièrement avantageuse notamment lorsque l'inoculum de départ pour démarrer la réaction exothermique de production de biomasse consiste en des levures sèches actives (LSA).

Dans un mode de réalisation préférentiel, le dispositif 1 selon l'invention comporte également un moyen de mesure de composition du gaz 7 à la sortie de la cuve de propagation 2, dans l'optique d'évaluer notamment la production de dioxyde de carbone CO₂ et d'oxygène O₂ au sein de ladite cuve de propagation 2.

La mesure du volume des gaz CO₂ et O₂ produits permet d'évaluer notamment le quotient respiratoire CO₂ produit/O₂consommé et, de ce fait, de contrôler quelle est la voie métabolique, respiration ou fermentation, utilisée par les levures pour dégrader le substrat carboné présent dans le milieu de culture 3. En effet, il a déjà été indiqué précédemment qu'il était préférable que les levures métabolisent les substances carbonées via la respiration pour favoriser la production de biomasse.

De manière toute particulière, tous les éléments du dispositif 1 selon l'invention, notamment les cuves 2 et 4, les tuyaux reliant lesdites cuves 2 et 4, la pompe d'alimentation 5 et autres éléments, sont équipés d'un système de nettoyage en place NEP (ou CIP pour « clean-in-place » en anglais).

Un tel système NEP permet avantageusement de nettoyer l'ensemble des éléments dudit dispositif 1, de manière automatique, et sans démontage, à l'aide d'un circuit d'eau ou de produit stérilisant parallèle.

Au moyen du dispositif 1 selon l'invention, pour une application dans la production de biomasse en quantité importante par des levures, les étapes suivantes peuvent être réalisées :
- avant de démarrer la réaction, l'ensemble des éléments dudit dispositif 1 est nettoyé au moyen du système NEP ;
- la source externe 31, consistant préférentiellement en du milieu réactionnel neuf, ou milieu de culture, est préparée et mise dans la cuve de stockage 4 ;
- une certaine proportion dudit milieu réactionnel est transférée au niveau de la cuve de propagation 2, via la pompe d'alimentation 5 ;
- les levures sont éventuellement réhydratées dans le récipient 6 de réhydratation des levures, par exemple lorsque l'inoculum de départ consiste en des levures sèches actives (LSA) ;
- dans la cuve de propagation 2, le milieu de culture 3 est inoculé ;
- le système d'alimentation automatique en source externe 31 refroidie, depuis la cuve de stockage 4 vers la cuve de propagation 2, est démarré ;
- après un certain temps, par exemple après 48 h de culture, il est possible de vérifier si une quantité correcte de gaz a été enregistrée par le moyen de mesure du gaz 7.

Si la quantité mesurée est correcte et correspond à ce qui est attendu, la culture peut être arrêtée et la biomasse peut être prélevée. Dans le cas contraire, il est envisageable d'évaluer le pH afin de déterminer si celui-ci a baissé, et attendre 24h supplémentaires avant de vérifier à nouveau la quantité de gaz relevée.

La cuve de stockage 4 peut être à nouveau remplie de source externe, c'est-à-dire de milieu de culture, et l'opération d'alimentation automatique redémarrée, après qu'une partie du volume de la culture de levure, par exemple la moitié, ait été prélevée dans la cuve de propagation 2.

## Revendications

1. Procédé de contrôle de la température d'une réaction exothermique, productrice de chaleur, ladite réaction ayant lieu dans un milieu réactionnel et faisant intervenir des cellules animales et/ou végétales et/ou des microorganismes, procédé dans lequel :
- on inocule ledit milieu réactionnel avec lesdites cellules ou lesdits microorganismes ;
- on permet à la réaction exothermique d'avoir lieu dans ledit milieu réactionnel ;
- on mesure une variation de la température due à la réaction exothermique, cette variation étant une augmentation;
- on ajoute progressivement audit milieu réactionnel, au cours de ladite réaction, au moins une source externe comprenant un substrat et/ou un réactif ;
ledit procédé étant **caractérisé par le fait que** l'on maintient, dans le milieu réactionnel, une température sensiblement constante en contrôlant au moins l'alimentation en source externe et la température de ladite source externe, l'alimentation en source externe et la température de cette dernière étant aptes à compenser la variation de la température mesurée due à la réaction exothermique, la source externe présentant une température basse, inférieure à la température dudit milieu, et apte à compenser la production de chaleur engendrée par ladite réaction exothermique, et en ce que, en plus de contrôler l'alimentation et la température de la source externe, on protège le milieu réactionnel de toute influence externe en termes d'apport ou de déperdition de chaleur.

2. Procédé de contrôle de la température d'une réaction exothermique selon la revendication précédente **caractérisé par le fait que** l'on maintient une température sensiblement constante dans le milieu réactionnel en contrôlant, en plus de la température de ladite source externe ajoutée audit milieu réactionnel, au moins la concentration en substrat et/ou en réactif dans ledit milieu réactionnel.

3. Procédé de contrôle de la température d'une réaction exothermique selon l'une quelconque des revendications précédentes **caractérisé par le fait que** ladite réaction exothermique permet la production de biomasse à partir de microorganismes, procédé dans lequel :
- on inocule un milieu réactionnel approprié, consistant en un milieu de culture liquide, avec lesdits microorganismes afin que ces derniers se multiplient dans ledit milieu de culture ;
- on mesure l'augmentation de la température due à la production de chaleur engendrée par la réaction exothermique de production de biomasse ;
- on ajoute progressivement, audit milieu de culture, au moins une source externe liquide comprenant un substrat carboné pour lesdits microorganismes ;
- on maintient, tout au long de la réaction de production de biomasse, une température sensiblement constante dans ledit milieu de culture en ajoutant une source externe liquide présentant une température basse apte à compenser ladite production de chaleur engendrée par ladite réaction de production de biomasse.

4. Procédé de contrôle de la température d'une réaction exothermique selon la revendication précédente dans lequel lesdits microorganismes consistent en des levures *Saccharomyces cerevisiae.*

5. Procédé de contrôle de la température d'une réaction exothermique selon la revendication précédente dans lequel le milieu de culture liquide est un milieu de culture hydroalcoolique.

6. Procédé de contrôle de la température d'une réaction exothermique selon la revendication précédente dans lequel le milieu de culture hydroalcoolique est supplémenté en glucides.

7. Dispositif (1) pour la mise en œuvre du procédé de contrôle de la température d'une réaction exothermique selon l'une quelconque des revendications précédentes comportant :
- au moins une cuve de propagation (2) comprenant un milieu réactionnel (3) inoculé avec des cellules animales et/ou végétales et/ou des microorganismes et au moins des moyens de mesure de la température de réaction à l'intérieur de ladite cuve de propagation (2) ;
- un centre (10) de collecte des données mesurées et de contrôle de ladite température de réaction ;
- au moins une cuve de stockage (4) comprenant une source externe (31), qui comprend un substrat et/ou un réactif, et étant reliée à ladite cuve de propagation (2) par un moyen de transfert (5) apte à être activé par ledit centre de contrôle (10) pour acheminer progressivement la source externe (31) depuis ladite cuve de stockage (4) vers ladite cuve de propagation (2) ;
ledit dispositif étant **caractérisé par le fait que** ladite cuve de propagation (2) est isolée thermiquement et que ladite cuve de stockage (4) est thermorégulée de manière à contrôler la température de la source externe (31) transférée vers ladite cuve de propagation (2), ladite cuve (2) étant réfrigérée via des moyens de refroidissement et maintenue à basse température au moyen d'une double enveloppe ou d'un circuit de refroidissement, l'ajout de la source externe (31) à une température basse étant apte à compenser la production de chaleur et une augmentation de température dans la cuve de propagation (2).

## Patentansprüche

1. Verfahren zum Steuern der Temperatur einer wärmeerzeugenden exothermen Reaktion, wobei die besagte Reaktion in einem Reaktionsmedium stattfindet, in dem tierische und/oder pflanzliche Zellen und/oder Mikroorganismen einbezogen sind, bei welchem Verfahren:
- das besagte Reaktionsmedium mit den besagten Zellen oder den besagten Mikroorganismen inokuliert wird;
- die exotherme Reaktion erlaubt wird, in dem besagten Reaktionsmedium stattzufinden;
- eine Temperaturänderung aufgrund der exothermen Reaktion gemessen wird, wobei diese Änderung eine Erhöhung ist;
- dem besagten Reaktionsmedium während der besagten Reaktion mindestens eine externe Quelle, die ein Substrat und/oder ein Reagenz umfasst, allmählich hinzugefügt wird;
wobei das besagte Verfahren **dadurch gekennzeichnet ist, dass** in dem Reaktionsmedium eine im wesentlichen konstante Temperatur aufrechterhalten wird, indem mindestens die Zufuhr einer externen Quelle und die Temperatur dieser Letzteren gesteuert werden, wobei die Zufuhr einer externen Quelle und die Temperatur dieser Letzteren in der Lage sind, die Änderung der gemessenen Temperatur aufgrund der exothermen Reaktion auszugleichen, wobei die externe Quelle eine niedrige Temperatur aufweist, die niedriger als die Temperatur des besagten Mediums ist, und in der Lage ist, die durch die exotherme Reaktion erzeugte Wärmeerzeugung auszugleichen, und dadurch, dass zusätzlich zum Steuern der Zufuhr und der Temperatur der externen Quelle das Reaktionsmedium vor jeglichen externen Einfluss hinsichtlich Wärmezufuhr oder Wärmeverlust geschützt wird.

2. Verfahren zum Steuern der Temperatur einer exothermen Reaktion nach dem vorhergehenden Anspruch, **dadurch gekennzeichnet, dass** eine im wesentlichen konstante Temperatur im Reaktionsmedium aufrechterhalten wird, indem zusätzlich zu der Temperatur der besagten externen Quelle, die dem Reaktionsmedium hinzugefügt wird, mindestens die Konzentration des Substrats und/oder des Reagens in dem besagten Reaktionsmedium gesteuert wird.

3. Verfahren zum Steuern der Temperatur einer exothermen Reaktion nach einem der vorhergehenden Ansprüche, **dadurch gekennzeichnet, dass** die exotherme Reaktion die Erzeugung von Biomasse aus Mikroorganismen erlaubt, bei welchem Verfahren:
- ein geeignetes Reaktionsmedium, das aus einem flüssigen Kulturmedium besteht, mit den besagten Mikroorganismen inokuliert wird, damit sich die Letzteren in dem Kulturmedium vermehren;
- die Temperaturerhöhung aufgrund der Wärmeerzeugung, die durch die exotherme Reaktion der Biomasseproduktion erzeugt wird, gemessen wird;
- dem besagten Kulturmedium mindestens eine externe Flüssigkeitsquelle, die ein kohlenstoffhaltiges Substrat für die besagten Mikroorganismen umfasst, allmählich hinzugefügt wird;
- während der gesamten Biomasseproduktionsreaktion eine im Wesentlichen konstante Temperatur in dem besagten Kulturmedium aufrechterhalten wird, indem eine, eine niedrige Temperatur aufweisende externe Flüssigkeitsquelle hinzugefügt wird, die geeignet ist, die besagte durch die Biomasseproduktionsreaktion erzeugte Wärmeerzeugung auszugleichen.

4. Verfahren zum Steuern der Temperatur einer exothermen Reaktion nach dem vorhergehenden Anspruch, bei dem die besagten Mikroorganismen aus *Saccharomyces cerevisiae*-Hefen bestehen.

5. Verfahren zum Steuern der Temperatur einer exothermen Reaktion nach dem vorhergehenden Anspruch, bei dem das flüssige Kulturmedium ein hydroalkoholisches Kulturmedium ist.

6. Verfahren zum Steuern der Temperatur einer exothermen Reaktion nach dem vorhergehenden Anspruch, bei dem das hydroalkoholische Kulturmedium mit Kohlenhydraten ergänzt wird.

7. Vorrichtung (1) zum Durchführen des Verfahrens zum Steuern der Temperatur einer exothermen Reaktion nach irgendeinem der vorhergehenden Ansprüche, umfassend:
- mindestens einen Ausbreitungsbehälter (2), der ein Reaktionsmedium (3) umfasst, das mit tierischen und/oder pflanzlichen Zellen und/oder Mikroorganismen inokuliert ist, und mindestens Mittel zum Messen der Reaktionstemperatur innerhalb des besagten Ausbreitungsbehälters (2);
- ein Zentrum (10) zum Sammeln der gemessenen Daten und zum Steuern der Reaktionstemperatur;
- mindestens einen Lagerbehälter (4), der eine externe Quelle (31) umfasst, die ein Substrat und/oder ein Reagenz umfasst, und der mit dem Ausbreitungsbehälter (2) über ein Übertragungsmittel (5) verbunden ist, das in der Lage ist, von dem Steuerzentrum (10) aktiviert zu werden, um die externe Quelle (31) allmählich von dem Lagerbehälter (4) zu dem Ausbreitungsbehälter (2) zu befördern;
wobei die Vorrichtung **dadurch gekennzeichnet ist, dass** der besagte Ausbreitungsbehälter (2) wärmeisoliert ist und dass der Lagerbehälter (4) wärmereguliert ist, sodass die Temperatur der zu dem Ausbreitungsbehälter (2) übertragenen externen Quelle (31) gesteuert wird, wobei der Ausbreitungsbehälter (2) über Kühlmittel gekühlt wird und mittels eines Doppelmantels oder eines Kühlkreislaufs auf niedriger Temperatur gehalten wird, wobei die Hinzufügung der externen Quelle (31) auf niedriger Temperatur in der Lage ist, die Wärmeerzeugung und einen Temperaturanstieg in dem Ausbreitungsbehälter (2) auszugleichen.

## Claims

1. Method for controlling the temperature of a heat-producing exothermic reaction, said reaction taking place in a reaction medium and involving animal and/or plant cells and/or microorganisms, method in which:
- said reaction medium is inoculated with said cells or said microorganisms;
- the exothermic reaction is allowed to take place in said reaction medium;
- a change in temperature due to the exothermic reaction is measured, this variation being an increase;
- to said reaction medium is gradually added, during said reaction, at least one external source comprising a substrate and/or a reagent;
said process being **characterized in that** a substantially constant temperature is maintained in the reaction medium by controlling at least the supply of an external source and the temperature of said external source, the supply of an external source and the temperature of the latter being capable of compensating for the variation in the measured temperature due to the exothermic reaction, the external source having a low temperature, lower than the temperature of said medium, and capable of compensating for the production of heat generated by said exothermic reaction, and **in that**, in addition to controlling the supply and the temperature of the external source, the reaction medium is protected from any external influence in terms of addition or loss of heat.

2. Method for controlling the temperature of an exothermic reaction according to the preceding claim, wherein a substantially constant temperature is maintained in the reaction medium by controlling, in addition to the temperature of said external source added to said reaction medium, at least the concentration of substrate and/or of reagent in said reaction medium.

3. Method for controlling the temperature of an exothermic reaction according to any one of the preceding claims, wherein said exothermic reaction permits the production of biomass from microorganisms, process in which:
- an appropriate reaction medium, consisting of a liquid culture medium, is inoculated with said microorganisms in order for the latter to multiply in said culture medium;
- the increase in temperature due to the production of heat generated by the exothermic reaction of biomass production is measured;
- to said culture medium is gradually added at least one liquid external source comprising a carbon-containing substrate for said microorganisms;
- throughout the biomass production reaction is maintained a substantially constant temperature in said culture medium by adding a liquid external source having a low temperature capable of compensating for said heat production generated by said biomass production reaction.

4. Method for controlling the temperature of an exothermic reaction according to the preceding claim, wherein said microorganisms consist of *Saccharomyces cerevisiae* yeasts.

5. Method for controlling the temperature of an exothermic reaction according to the preceding claim, wherein the liquid culture medium is a hydro-alcoholic culture medium.

6. Method for controlling the temperature of an exothermic reaction according to the preceding claim, wherein the hydro-alcoholic culture medium is supplemented with carbohydrates.

7. Device (1) for implementing the process for controlling the temperature of an exothermic reaction according to any one of the preceding claims, comprising:
- at least one propagation tank (2) comprising a reaction medium (3) inoculated with animal and/or plant cells and/or microorganisms and at least means for measuring the reaction temperature inside said propagation tank (2);
- a center (10) for collecting the measured data and for controlling said reaction temperature;
- at least one storage tank (4) comprising an external source (31), which comprises a substrate and/or a reagent, and being connected to said propagation tank (2) by a transfer means (5) capable of being activated by said control center (10) to progressively convey the external source (31) from said storage tank (4) to said propagation tank (2);
said device being **characterized in that** said propagation tank (2) is thermally insulated and that said storage tank (4) is temperature controlled so as to control the temperature of the external source (31) transferred to said propagation tank (2), said tank (2) being refrigerated via cooling means and kept at low temperature by means of a double jacket or a cooling circuit, the addition of the external source (31) at a low temperature being capable of compensating for the production of heat and an increase in temperature in the propagation tank (2).
